# EUROPEAN PATENT APPLICATION

(11) **EP 4 818 181 A1**
(43) Date of publication of application: **07.10.2026**
(21) Application number: 23942228.0
(22) Date of filing: 05.12.2023
(51) Int. Cl.: C07F 5/02, A61K 47/24, A61K 31/7088

(54) **OXIDATION-RESPONSIVE CATIONIC WATER-SOLUBLE PILLARARENE, AND PREPARATION METHOD AND USE**

(30) Priority: 01.12.2023 CN 202311631179
(71) Applicant: Zhejiang University, Hangzhou, Zhejiang 310027 (CN)
(72) Inventor: LIN, Xianfeng, Hangzhou, Zhejiang 310027 (CN); SHANGGUAN, Liqing, Hangzhou, Zhejiang 310027 (CN); ZHU, Yiwei, Hangzhou, Zhejiang 310027 (CN); GU, Chenhui, Hangzhou, Zhejiang 310027 (CN)
(74) Representative: JD&P Patent Attorneys Joanna Dargiewicz & Partners
(86) International application number: PCT/CN2023/136609
(87) International publication number: WO 2025/112086

(57) **Abstract**

Disclosed are an oxidation-responsive water-soluble cationic pillararene, and a preparation method and application thereof, the oxidation-responsive water-soluble cationic pillararene is a cyclic small-molecule nucleic acid vector, and is obtained by quaternization reaction of 4-methylborate and tertiary amine-modified pillararene. According to the invention, a delivery vector with a high positive charge density is obtained through small molecule synthesis, the delivery vector is capable of being tightly combined with negatively charged nucleic acid substances to form a nano-composite, and after the nano-composite enters cells, vector positive charges fall off to release the nucleic acid substances in an oxidation environment, and nucleic acid information is efficiently expressed. The delivery vector has the characteristics of simplicity and convenience in synthesis, high delivery efficiency and good biological safety, and has a good application prospect.

## Description

### TECHNICAL FIELD

The present invention belongs to the scientific field of organic synthesis biological medicines, and particularly relates to an oxidation-responsive water-soluble cationic pillararene, a preparation method thereof and an application thereof as a nucleic acid delivery vector.

### BACKGROUND OF THE PRESENT INVENTION

Among many existing cancer treatment methods, gene therapy can achieve therapeutic effects by directly repairing and improving gene defects, which are difficult to be achieved by traditional drugs. Due to the development and completion of the human genome project, the identification of many pathogenic genes provides a broader idea and method for gene therapy, so that researchers can explore innovative and efficient gene therapy strategies in a more targeted way. The research and development of gene delivery vector is a key link.

Gene delivery technology mainly comprises three categories of viral vector, non-viral vector and physical transfection technology, and compared with the other two categories of gene delivery methods, the non-viral vector is easier to overcome the defects of vector itself, such as pathogenicity and immunogenicity, and has high biological safety and low cost, thus showing significant advantages. At present, the main research challenges in the development of non-viral vector mainly comprise: 1. the improvement of in-vivo circulation stability of gene delivery system; 2. an efficient focus targeting ability; and 3. the breakthrough of bottleneck of low transfection efficiency. Therefore, it is a powerful way to break through the challenge of research on the non-viral gene vector to improve the performance of the gene delivery system by the innovative optimization of vector structure.

The recognition of host and guest based on macrocyclic molecules is one of the directions with great research and development potential in supramolecular chemistry. The recognition of host and guest between macrocyclic molecules and guest molecules by various weak interactions provides new, simpler and more efficient idea and method for researchers to construct stimulus-responsive materials and innovative materials. Because of unique structural properties, pillararenes have shown good application prospects in researches and become the leader among the new generation of macrocyclic entities. The repeating unit 1,4-dimethoxybenzene of pillararene is linked to 2 and 5 positions through methylene bridged bonds to form a symmetrical pillar structure, so that the pillararene has more unique properties and advantages: (a) a molecular cavity is rich in electrons, and it is easy to form a novel host-guest complex; (b) a molecular structure is easy to be chemically synthesized and modified, and a pillararene molecule in a required structure may be obtained through simple and efficient chemical reaction; and (c) there is rich stimulus responsiveness, which may be achieved not only by dynamically reversible host-guest complex interaction, but also by introducing specific responsive modifying groups. These features are also the key to realize the wide application and achievements of functional materials based on pillararene construction in various fields.

Therefore, the present invention provides a preparation method of an oxidation-responsive water-soluble cationic pillararene, in which the pillararene can be tightly combined with a negatively charged nucleic acid through electrostatic interaction. Meanwhile, in combination with a physiological microenvironment characteristic that cancer, injury, inflammation and other lesions all have a large number of free radicals of reactive oxygen species (ROS), the pillararene can effectively load a nucleic acid drug, and at the same time, after the pillararene reaching a lesion site, the positive electricity of the pillararene is removed by the oxidization and falling off of borate to release the nucleic acid drug, so as to improve the efficient delivery and release of the nucleic acid drug in gene therapy.

To sum up, according to the oxidation-responsive water-soluble cationic pillararene in the present invention, a charge change is triggered by an ROS microenvironment in focus cells to realize the release of the nucleic acid-loaded drug. At present, there is no report about this oxidation-responsive water-soluble cationic pillararene, and there is no report about a preparation method and application thereof.

### SUMMARY OF THE PRESENT INVENTION

Aiming at the defects in the prior art, the present invention provides an application of an oxidation-responsive water-soluble cationic pillararene as a nucleic acid vector in gene therapy. The pillararene prepared in the present invention has the advantages and features of efficient loading and release, good biocompatibility and the like, can form a nano-composite with DNA, RNA and other short-chain nucleic acids, and has higher transfection efficiency.

An oxidation-responsive water-soluble cationic pillararene comprises the following structure: 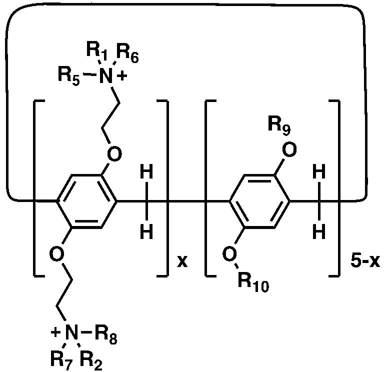 in the above formula:
X is an integer from 1 to 4, R₁ and R₂ are independently the following segments respectively, and R₃ and R₄ are independently H and C1-C6 alkyl or acyl respectively; 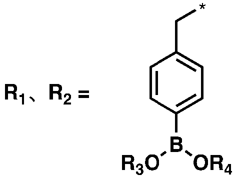
R₅ and R₆, and R₇ and R₈ are independently H and C1-C6 alkyl or aryl respectively;
R₇ and R₉ are independently H and C1-C20 alkyl or aryl respectively; and
anions are bromide ions or chloride ions.

Preferably, reaction monomers 1-(2-haloethoxy)-4-methoxybenzene and 4-alkoxymethyl(ethyl)oxybenzene react together under the condition of Lewis acid catalyst to obtain the copolymerized pillar[5]arene obtained by copolymerization according to reaction monomer molar ratios of 2: 3 and 1: 4, then react with dimethylamine and diethylamine to obtain a copolymerized pillar[5]arene substituted with tertiary amine, and then react with boric acid benzyl bromide, borate benzyl bromide, boric acid benzyl chloride or borate benzyl chloride to obtain the pillararene compound.

Preferably, the pillararene is a copolymerized pillar[5]arene, and a copolymerization ratio is 1: 4, that is, x = 1 or 4, and 2: 3, that is, x = 2 or 3.

Preferably, the copolymerized pillar[5]arene comprises a pillar[5]arene obtained by copolymerization cyclization reaction according to a reaction monomer molar ratio of 2: 3, with an obtained product x = 2 or 3; and according to a reaction monomer molar ratio of 1: 4, with an obtained product x = 1 or 4.

Preferably, the above R₃ and R₄ are boric acid, methyl borate, ethyl borate or pinacol borate ester.

Preferably, the above R₅, R₆, R₇ and R₈ are methyl or ethyl.

Preferably, the above R₉ is methyl or ethyl, and the R₁₀ is alkyl or aryl with a C number greater than 6, and more preferably n-alkyl.

Preferably, the above compound may be prepared by the following method:

1-(2-haloethoxy)-4-methoxybenzene and 4-alkoxymethyl(ethyl)oxybenzene react together under the condition of Lewis acid catalyst to obtain the copolymerized pillar[5]arene obtained by copolymerization according to a monomer ratio of 1: 4, then react with dimethylamine and diethylamine to obtain a copolymerized pillar[5]arene substituted with tertiary amine, and then react with boric acid benzyl bromide, borate benzyl bromide, boric acid benzyl chloride or borate benzyl chloride to obtain the pillararene compound.

A preparation method thereof is as shown in the following formula: 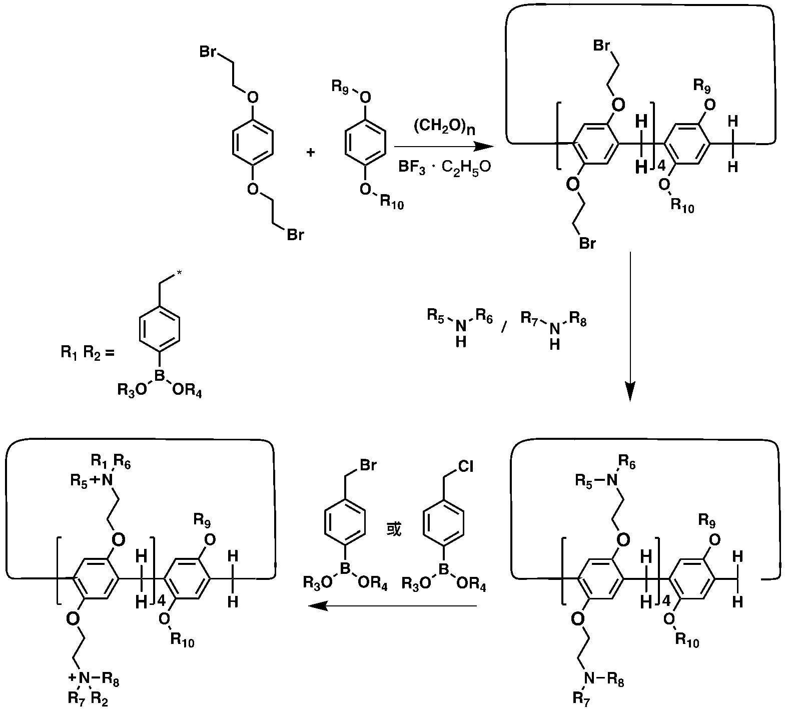 a positive charge of quaternary amine salt of the oxidation-responsive water-soluble cationic pillararene of the present invention can fall off through oxidation reaction with active oxygen species such as hydrogen peroxide to be converted into electrically neutral tertiary amine, and a reaction formula of this process is as follows:

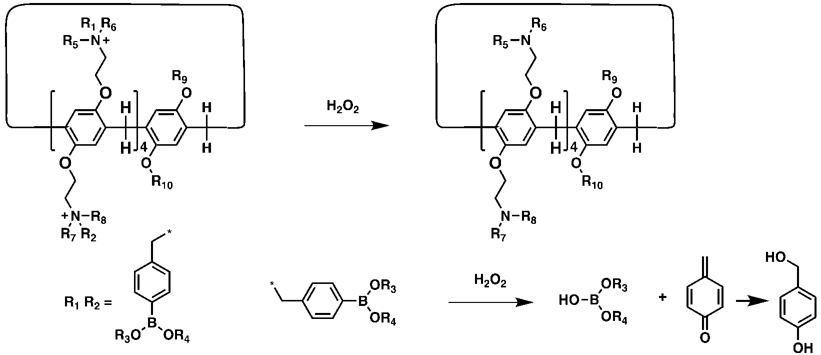

The present invention further provides an application of the oxidation-responsive water-soluble cationic pillararene above in delivery of DNA, RNA and other short-chain nucleic acids.

Compared with the prior art, the present invention has the following beneficial effects.
(1) The oxidation-responsive water-soluble cationic pillararene prepared in the present invention belongs to a small-molecule gene delivery vector, which not only has the characteristics of high charge density and high nucleic acid loading efficiency, but also has simple structure and cheap synthesis, and has higher stability and reproducibility compared with a traditional polymer nucleic acid vector.
(2) The nucleic acid vector of the oxidation-responsive water-soluble cationic pillararene prepared in the present invention can realize effective nucleic acid release through reaction with reactive oxygen species, so as to avoid the problem that a traditional quaternization vector is difficult to relay because of being too tightly linked to a nucleic acid to cause the decrease of transfection efficiency.
(3) The nucleic acid vector of the oxidation-responsive water-soluble cationic pillararene prepared in the present invention shows higher transfection efficiency in cells, has lower cytotoxicity compared with gold standard PEI for gene transfection, and has good biocompatibility.

### DESCRIPTION OF THE DRAWINGS

FIG. 1 is a ¹HNMR spectrogram of a compound 1 in Embodiments 1, 2 and 3 under oxidation conditions;
FIG. 2 shows particle sizes and Zeta potentials of nano-composites with different N/P formed by combining a compound 1 in Embodiment 4 of the present invention with a plasmid DNA;
FIG. 3 is a gel retardation electrophoretogram of the nano-composites with different N/P formed by combining the compound 1 in Embodiment 4 of the present invention with the plasmid DNA;
FIG. 4 is a gel retardation electrophoretogram under oxidation conditions of the nano-composite formed by combining the compound 1 in Embodiment 4 of the present invention with the plasmid DNA;
FIG. 5A shows particle sizes under oxidation conditions of the nano-composite formed by combining the compound 1 in Embodiment 4 of the present invention with the plasmid DNA; and FIG. 5B is a change diagram of corresponding Zeta potentials;
FIG. 6 is a transmission electron micrograph of the nano-composite formed by combining the compound 1 in Embodiment 4 of the present invention with the plasmid DNA;
FIG. 7 is a cytotoxicity diagram of a compound 1 in Embodiment 5 of the present invention at different concentrations;
FIG. 8 is an effect diagram of cell transfection under different N/P conditions of a nano-composite formed by combining a compound 1 in Embodiment 6 of the present invention with the plasmid DNA;
FIG. 9 is an effect diagram of cell transfection under different N/P conditions of a nano-composite formed by combining a compound 1 in Embodiment 7 of the present invention with an mRNA; and
FIG. 10 is an effect diagram of cell transfection under oxidation conditions of a nano-composite formed by combining a compound 1 in Embodiment 8 of the present invention with the DNA.

### DETAILED DESCRIPTION OF PREFERRED EMBODIMENTS

The present invention provides some specific embodiments, but the present invention is not limited by these embodiments.

### Embodiment 1:

### Synthesis of compound 1

1-methoxy-4-hexadecylbenzene (1.74 g, 5.00 mmol) and 4-bis(2-bromoethoxy)benzene (6.48 g, 20.0 mmol) were placed in 80 mL of 1,2-dichloroethane, then added with boron trifluoride diethyl ether (3.20 mL, 25 mM), and stirred at room temperature for 2 hours. After reaction, the reaction solution was poured into methanol to separate out a large number of white solids and filtered to obtain a precipitate, and the precipitate was dissolved in dichloromethane and then filtered again to remove insoluble substances. The dichloromethane solution was washed with water twice to obtain an organic phase, and the organic phase was dried with anhydrous sodium sulfate and then spin-dried to obtain a crude product. The crude product was subjected to column chromatography, a mobile phase was petroleum ether/ethyl acetate = 50: 1 (R_{f} = 0.50), and a product compound 3 was obtained by spin drying, which was a white powdery solid (0.85 g, 10%).

The compound 3 (1.64 g, 1.00 mmol) and excess diethylamine (7.50 g, 100 mmol) were added into 100 mL of absolute ethanol, and heated and stirred at 80°C for reflux reaction for 24 hours. After reaction, the solvent was removed by rotary evaporation, and 200 mL of 1 M sodium hydroxide solution was poured into the mixture and stirred for 1 hours. Subsequently, the reaction solution was fully extracted with ethyl acetate, and an organic phase was spin-dried to obtain a dark yellow oily compound 2 (1.55 g, 98%).

The compound 2 (0.49 g, 0.30 mM) and 4-(bromomethyl)benzeneboronic acid pinacol ester (0.78 g, 2.64 mmol) were dissolved in 25 mL of acetonitrile, and heated and stirred at 75°C for reflux reaction for 24 hours. After reaction, the mixed solution obtained from the reaction was concentrated to 2.0 mL, added with excessive ether, and filtered to collect a white precipitate separated out, and the precipitate was fully washed with ether, and dried in a vacuum oven to obtain a white precipitate, which was a product compound 1 (0.98 g, 81.6%).

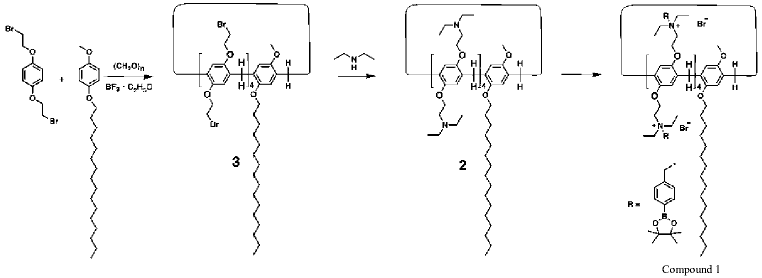

Structure detection data of the compound 1 were as follows:
¹H NMR (400 MHz, D₂O, 298 K) δ (ppm): 7.80-7.73 (m, 16H), 7.54-7.45 (m, 16H), 6.97-6.63 (m, 10H), 4.79-4.58 (m, 16H), 4.48-4.38 (m, 16H), 4.30-4.25 (t, 2H), 3.86-3.77 (m, 16H), 3.68 (s, 3H), 3.65-3.50 (s, 10H), 3.50-3.42 (m, 32H), 1.46-1.39 (m, 48H), 1.23 (s, 96H), 0.71-0.41 (m, 31H). ¹³C NMR (600 MHz, CD₃OD, 298 K) δ (ppm): 151.45, 136.54, 135.77, 133.44, 133.01, 131.50, 117.47, 85.54, 75.81, 70.92, 63.47, 58.05, 55.57, 33.05, 30.79, 30.46, 25.27, 25.03, 23.72, 14.47, 8.96. HR-MS: [M-8Br]⁸⁺ m/z determined as 422.8757, [M-7Br]⁷⁺ m/z determined as 494.5405. A melting point was 162.4°C to 162.9°C.

### Embodiment 2:

### H₂O₂ responsiveness of compound

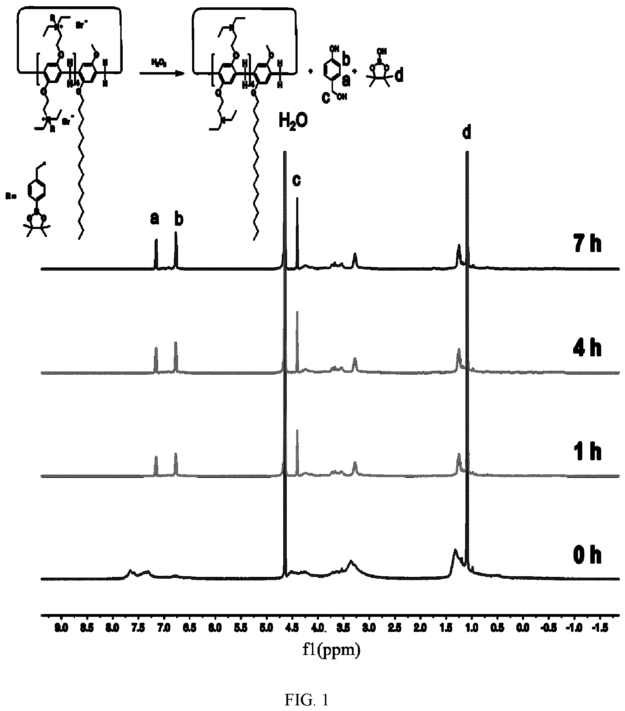

A certain amount of compound 1 was dissolved in D₂O (1 mM), and dropwise added with a small amount of hydrogen peroxide to reach a final concentration of 10 mM. Under oxidation conditions, the compound 1 reacted quickly and produced quinone, and the quinone was converted into p-hydroxybenzyl alcohol in water. In this process of change, corresponding proton peaks a, b and c could be observed in ¹H NMR, which proved that the compound 1 made a redox response. Detection results were as shown in FIG. 1.

### Embodiment 4:

### Preparation and characterization of nano-composite of compound 1 and plasmid DNA

A certain amount of compound 1 was dissolved in an HEPES buffer solution (pH = 7.4, 10 mM) at a concentration of 2.0 mg/ml, and a plasmid DNA was also diluted to a concentration of 40 µg/ml with the HEPES buffer solution at the same time. After the compound was diluted to a corresponding concentration according to a corresponding N/P molar ratio, the compound was quickly added into the plasmid DNA solution according to a volume ratio of 1: 1, and vortexed and vibrated for 30 seconds and then allowed to stand for 30 minutes, so as to obtain a series of nano-composites with different N/P.

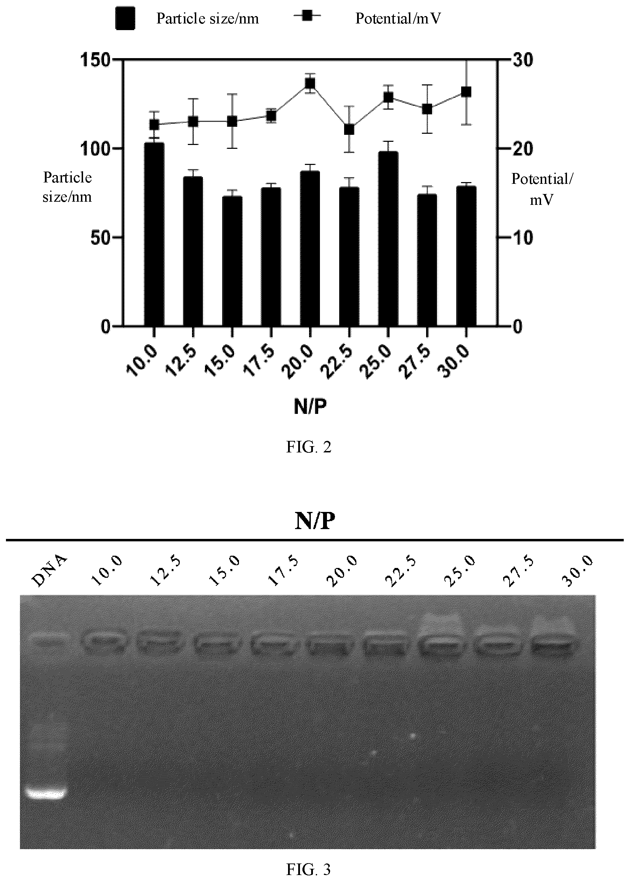

Particle size and potential of nano-composite: a proper amount of the series of nano-composites with different N/P prepared above were placed in a sample pool, particle sizes and Zeta potentials of nano-composite solutions with different N/P were measured by using a dynamic light scattering instrument, and each sample was repeatedly experimented for 3 times to obtain an average value. As shown in FIG. 2, the particle sizes of the nano-composites were about 70 nm to 100 nm, and the zeta potentials were 20 mV to 30 mV.

Gel retardation experiment of nano-composite: 1.0% agarose gel (containing 2 µg/ml gelred) was prepared and placed in a 1×TAE buffer solution, and 20 µL of the nano-composites with different N/P to be tested was added into a gel pore respectively. 20 µL of pure plasmid DNA at the same concentration was used as a control, and a voltage of 120 mV was applied to electrophoresis for 30 minutes. After electrophoresis, the gel was placed in a gel imaging system to shoot, and results were as shown in FIG. 3, which showed that the compound 1 wrapped with the DNA could well retard the migration of DNA, thus effectively protecting the DNA during gene delivery.

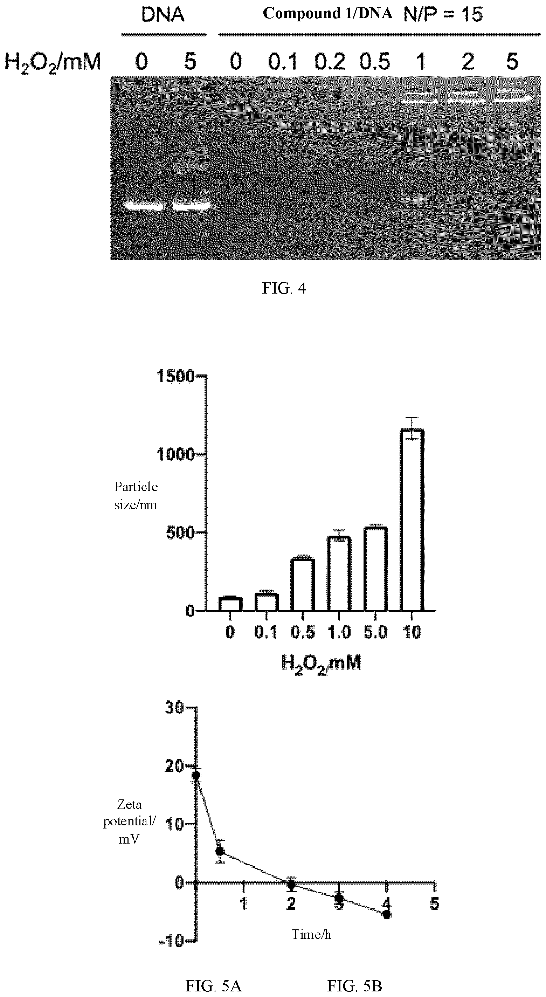

Gel retardation experiment of nano-composite under oxidation conditions: a nano-composite with N/P of 15 was incubated in H₂O₂ solutions with different concentrations at 37°C for 30 minutes, and then, in the same way, 1.0% agarose gel (containing 2 µg/ml gelred) was prepared and placed in a 1×TAE buffer solution, and 20 µL of the incubated nano-composites was added into a gel pore respectively. 20 µL of pure plasmid DNA at the same concentration was used as a control, and a voltage of 120 mV was applied to electrophoresis for 30 minutes. After electrophoresis, the gel was placed in a gel imaging system to shoot, and results were as shown in FIG. 4, which showed that the nano-composites could effectively release the DNA under oxidation conditions.

Changes of particle size and potential of nano-composite under oxidation conditions: the nano-composite with N/P of 15 was incubated in H₂O₂ solutions with different concentrations at 37°C for 30 minutes, a proper amount of the incubated nano-composites were placed in a sample pool, particle sizes of nano-composite solutions with different N/P were measured by using a dynamic light scattering instrument, and each sample was repeatedly experimented for 3 times to obtain an average value. The nano-composite with N/P of 15 was incubated in 1.0 mM H₂O₂ at 37°C, a proper amount of sample was taken in different time points and placed in a sample pool, point positions were measured by using a dynamic light scattering instrument, and each sample was repeatedly experimented for 3 times to obtain an average value. Results were as shown in FIG. 5A and FIG. 5B, with the increase of concentration of hydrogen peroxide, the particle size of the nano-composite was gradually increased, and a tight bond was dissociated; and with the extension of time, a surface potential of the nano-composite was changed from positive to negative, and an electrostatic attraction with the negatively charged DNA disappeared.

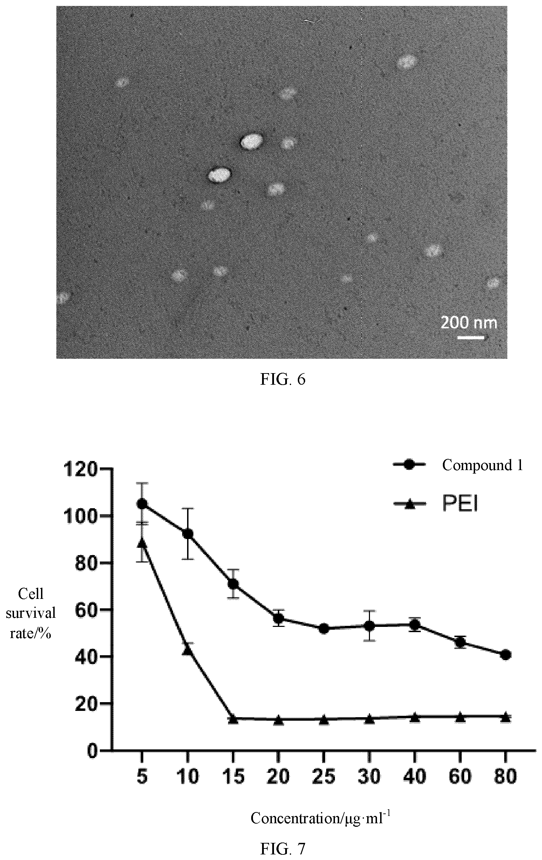

Projection electron microscope observation experiment of nano-composite: the nano-composite with N/P of 15 was dropwise added onto a 300-mesh copper mesh, and then negatively stained with phosphotungstic acid. After the liquid was suck-dried with an edge of filter paper, the nano-composite was naturally dried at room temperature, and then the nano-composite on the copper mesh was observed with a transmission electron microscope. As shown in FIG. 6, the compound 1 was combined with the plasmid DNA to form a nearly spherical nano-particle with regular morphology and a particle size of about 80 nm, which was basically consistent with the results of dynamic light scattering detection.

### Embodiment 5:

Cytotoxicity experiment of compound 1: the cytotoxicity evaluation of the compound 1 was characterized by a CCK8 kit. Cells were cultured in vectors of compounds 1 at different concentrations, and a traditional polymer gene vector PEI was used as a control. The cells were incubated for 48 hours, and after culture, the culture medium was discarded and a diluted CCK8 reagent was added to continuously incubate the cells for 1 hour to 2 hours. A light absorbance value at a wavelength of 450 nm was measured with a microplate reader, which was compared with that of the control group, and a survival ratio of the cells was calculated. Results were as shown in FIG. 7, with the increase of concentration, the survival rate of the cells under the treatment of PEI was decreased sharply, while the survival rate of the cells under the treatment of the compound 1 was decreased slowly, which was significantly higher than that of the PEI, thus proving that the compound 1 had higher biological safety in the measured concentration range.

### Embodiment 6:

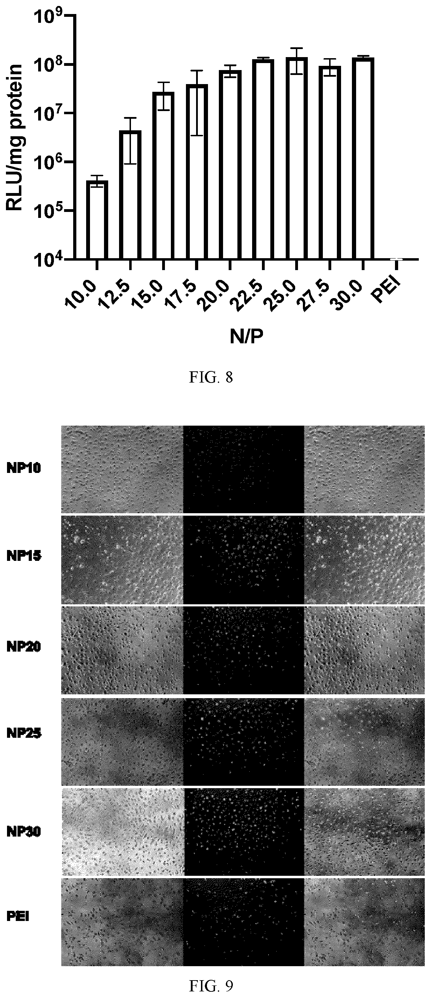

Luciferase gene transfection experiment: A549 cells were cultured in a 96-well plate with a cell density of 15000 cells/well and 200 µL of culture medium in each well, and then cultured in an incubator with 5% CO₂ and 95% humidity at 37°C for 24 hours, and then the culture medium was discarded and replaced with a fresh serum-free medium. The compound 1 was combined with a luciferase gene plasmid to form nano-composites with different N/P, added into the culture medium, and incubated at 37°C for 4 hours, and then the culture medium wad discarded again and replaced with a fresh culture medium to continue culture for 48 hours. After culture, the culture medium was discarded and added with 20 µL of 1× cell lysate, and after lysis for 10 minutes, 5 µL of supernatant was taken and added with 20 µL of luciferase substrate. A chemiluminescence intensity was measured with a chemiluminescence detector, and a protein concentration was measured with a Bradford protein detection kit. Three repeated wells were measured in parallel for each group of data to obtain an average value, and the chemiluminescence intensity was normalized by the protein concentration to obtain a luminescence intensity per mg of protein (RLU/mg protein). Results were as shown in FIG. 8, and compared with the commonly used non-viral gene vector PEI, the transfection efficiency of the compound 1 was improved by 1 to 2 orders of magnitude, indicating that the compound 1 was a more efficient gene vector.

### Embodiment 7:

Green fluorescent protein mRNA transfection experiment: RAW264.7 cells were cultured in a glass-bottom culture dish with a radius of 15 mm, with a cell density of 25000 cells/dish, and added with 1.5 mL of culture medium, and the cells were cultured in an incubator with 5% CO₂ and 95% humidity at 37°C for 24 hours. Subsequently, the culture medium in the culture dish was replaced with a serum-free medium, and added with a composite of the prepared compound 1 and a green fluorescent protein mRNA to incubate at 37 °C for 4 hours, and then the culture medium was discarded again and replaced with a fresh culture medium to continuously culture for 48 hours. After culture, the expression of the green fluorescent protein was observed with a laser confocal microscope, and at an excitation wavelength of 488 nm and an emission wavelength of 510 nm to 540 nm, all photos were taken under an objective lens of 10 times and a light intensity of shooting for all samples was kept the same. Results were as shown in FIG. 9, and compared with the PEI, the compound 1 loaded with the mRNA could realize higher transfection efficiency.

### Embodiment 8:

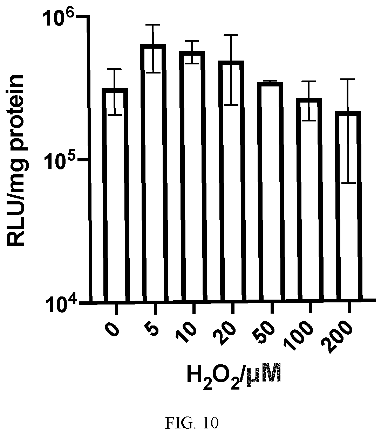

Cell transfection experiment of nano-composite under oxidation conditions: A549 cells were cultured in a 96-well plate, with a cell density of 15000 cells/well and 200 µL of culture medium in each well, and cultured in an incubator with 5% CO₂ and 95% humidity at 37°C for 24 hour. The culture medium was discarded and replaced with a fresh culture medium, and the culture medium was added with H₂O₂ to prepare high-oxidation environment media at different concentrations (5 µM, 10 µM, 20 µM, 50 µM and 100 µM) to simulate a high oxidation microenvironment of tumors. A compound 1 and a luciferase gene plasmid were added for combination to form nano-composites with different N/P, and the nano-composites were added into the culture medium to incubate at 37 °C for 4 hours. The culture medium was discarded again and replaced with a fresh culture medium to continuously culture for 48 hours. After culture, the culture medium was discarded and added with 20 µL of 1× cell lysate, and after lysis for 10 minutes, 5 µL of supernatant was taken and added with 20 µL of luciferase substrate. A chemiluminescence intensity was measured with a chemiluminescence detector, and a protein concentration was measured with a Bradford protein detection kit. Three repeated wells were measured in parallel for each group of data to obtain an average value, and the chemiluminescence intensity was normalized by the protein concentration to obtain a luminescence intensity per mg of protein (RLU/mg protein). Results were as shown in FIG. 10, which showed that the expression efficiency of the plasmid DNA loaded by the compound 1 was improved in the oxidation environment.

## Claims

1. An oxidation-responsive water-soluble cationic pillararene, **characterized by** comprising the following structure: 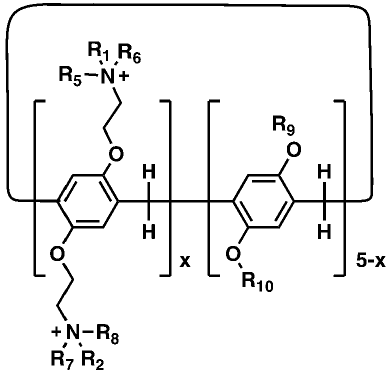 in the above formula:
X is an integer from 1 to 4, R₁ and R₂ are independently the following segments respectively, and R₃ and R₄ are independently H and C1-C6 alkyl or acyl respectively; 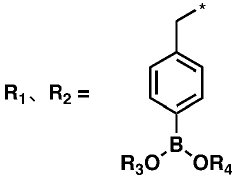
R₅ and R₆, and R₇ and R₈ are independently C1-C6 alkyl or aryl respectively;
R₉ and R₁₀ are independently H and C1-C20 alkyl or aryl respectively; and
anions are bromide ions or chloride ions.

2. The oxidation-responsive water-soluble cationic pillararene according to claim 1, **characterized in that** the pillararene is prepared by reacting a pillararene containing primary amino, secondary amino or tertiary amine with boric acid benzyl or borate benzyl.

3. The oxidation-responsive water-soluble cationic pillararene according to claim 2, **characterized in that** the pillararene is a copolymerized pillar[5]arene, and a copolymerization ratio is 1: 4, that is, x = 1 or 4, and 2: 3, that is, x = 2 or 3.

4. The oxidation-responsive water-soluble cationic pillararene according to claim 1 or 3, **characterized in that** the copolymerized pillar[5]arene comprises a pillar[5]arene obtained by copolymerization cyclization reaction according to a reaction monomer molar ratio of 2: 3, with an obtained product x = 2 or 3; and according to a reaction monomer molar ratio of 1: 4, with an obtained product x = 1 or 4.

5. The oxidation-responsive water-soluble cationic pillararene according to claim 1, 3 or 4, **characterized in that** R₅ and R₆, and R₇ and R₈ are methyl or ethyl, R₉ is methyl and ethyl, and R₁₀ is C6-20 alkyl or aryl.

6. The oxidation-responsive water-soluble cationic pillararene according to claim 1, 2, 3 or 4, **characterized in that** the pillararene compound is specifically as follows:
1-(2-haloethoxy)-4-methoxybenzene and 4-alkoxymethyl(ethyl)oxybenzene react together under the condition of Lewis acid catalyst to obtain the copolymerized pillar[5]arene obtained by copolymerization according to the molar ratios of 2: 3 and 1: 4, then react with dimethylamine and diethylamine to obtain a copolymerized pillar[5]arene substituted with tertiary amine, and then react with boric acid benzyl bromide, borate benzyl bromide, boric acid benzyl chloride or borate benzyl chloride to obtain the pillararene compound.

7. An application of an oxidation-responsive water-soluble cationic pillararene in delivery of nucleic acid substances, comprising DNA, RNA and other short-chain nucleic acids.

8. The preparation method of the oxidation-responsive water-soluble cationic pillararene according to claim 2, **characterized in that** the preparation method of the pillararene compound is specifically as follows:
1-(2-haloethoxy)-4-methoxybenzene and paraformaldehyde or trioxane react together under the condition of Lewis acid catalyst to obtain a cyclic pillar[n]arene, wherein n = 5 to 15, then react with dimethylamine and diethylamine to obtain a pillar[n]arene substituted with tertiary amine, and then react with boric acid benzyl bromide, borate benzyl bromide, boric acid benzyl chloride or borate benzyl chloride to obtain the pillararene compound.

9. The preparation method of the oxidation-responsive water-soluble cationic pillararene according to claim 2, wherein a structure of the water-soluble pillararene prepared by the method is as follows:
